(19) Europäisches Patentamt
European Patent Office
Office européen des brevets

(11) EP 2 875 357 B1

(12) **FASCICULE DE BREVET EUROPEEN**

(45) Date de publication et mention de la délivrance du brevet:
**07.09.2016 Bulletin 2016/36**

(21) Numéro de dépôt: **13744749.6**

(22) Date de dépôt: **16.07.2013**

(51) Int Cl.:
***G01N 33/574*** $^{(2006.01)}$ ***G01N 33/74*** $^{(2006.01)}$

(86) Numéro de dépôt international:
**PCT/FR2013/051700**

(87) Numéro de publication internationale:
**WO 2014/013180 (23.01.2014 Gazette 2014/04)**

(54) **METHODE THERANOSTIQUE BASEE SUR LA DETECTION DE DIMERES HER2-HER2**
THERANOSTISCHES VERFAHREN AUF BASIS DES NACHWEISES VON HER2-HER2-DIMEREN
THERANOSTIC METHOD BASED ON THE DETECTION OF HER2-HER2 DIMERS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.07.2012 FR 1256902**

(43) Date de publication de la demande:
**27.05.2015 Bulletin 2015/22**

(73) Titulaires:
- **Cisbio Bioassays**
**30200 Codolet (FR)**
- **Institut Gustave Roussy**
**94805 Villejuif Cédex (FR)**

(72) Inventeurs:
- **ANDRE, Fabrice**
**F-92330 Sceaux (FR)**
- **BAZIN, Hervé**
**F-30400 Villeneuve Les Avignon (FR)**
- **GARNERO, Patrick**
**F-30330 Gaujac (FR)**
- **MATHIS, Gérard**
**F-30200 Bagnols Sur Ceze (FR)**

(74) Mandataire: **Nevant, Marc et al**
**Cabinet Beau de Loménie**
**158, rue de l'Université**
**75340 Paris Cedex 07 (FR)**

(56) Documents cités:
**WO-A1-2009/086197**

- **N. GABORIT ET AL: "Time-resolved Fluorescence Resonance Energy Transfer (TR-FRET) to Analyze the Disruption of EGFR/HER2 Dimers: A NEW METHOD TO EVALUATE THE EFFICIENCY OF TARGETED THERAPY USING MONOCLONAL ANTIBODIES", JOURNAL OF BIOLOGICAL CHEMISTRY, vol. 286, no. 13, 1 avril 2011 (2011-04-01), pages 11337-11345, XP055021021, ISSN: 0021-9258, DOI: 10.1074/jbc.M111.223503 cité dans la demande & N Gaborit ET AL: "Time-resolved Fluorescence Resonance Energy Transfer (TR-FRET) to Analyze the Disruption of EGFR/HER2 Dimers: A NEW METHOD TO EVALUATE THE EFFICIENCY OF TARGETED THERAPY USING MONOCLONAL ANTIBODIES", Journal of Biological Chemistry, Supplementary information, 18 janvier 2011 (2011-01-18), XP055061426, Extrait de l'Internet: URL:http://www.jbc.org/content/suppl/2011/01/31/M111.223503.DC1/jbc.M111.223503-1.pd f [extrait le 2013-04-29]**
- **P Garnero ET AL: "Assessment of HER receptor oligomerization by Time-Resolved Fluorescence Resonance Energy transfer (TR-FRET): a potential new biomarker of anti-HER therapy response", , 29 juin 2010 (2010-06-29), XP055061393, Extrait de l'Internet: URL:http://www.htrf.com/sites/default/file s/ressources/Poster_Garnero_SBS2010_toolbo x.pdf [extrait le 2013-04-29]**
- **, 1 janvier 2009 (2009-01-01), XP055021123, Extrait de l'Internet: URL:http://www.htrf.com/technology/htrfthe ory/tr_basics/ [extrait le 2012-03-07]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 875 357 B1

- **ALEXANDRE HO-PUN-CHEUNG ET AL: "Quantification of HER Expression and Dimerization in Patients' Tumor Samples Using Time-Resolved Förster Resonance Energy Transfer", PLOS ONE, vol. 7, no. 7, 19 juillet 2012 (2012-07-19), page e37065, XP055061400, DOI: 10.1371/journal.pone.0037065**

## Description

L'invention concerne une méthode théranostique fondée sur la détection par une approche TR-FRET, c'est-à-dire par la mesure en temps résolu de la fluorescence émise lorsque deux molécules fluorescentes compatibles sont à proximité l'une de l'autre, d'homodimères constitués de deux protéines HER2 (ci-après « Her2-Her2 »). Cette méthode vise à déterminer si des patients atteints de cancer sont susceptibles de répondre à un traitement thérapeutique basé sur l'administration d'un anticorps spécifique de la protéine HER2, de type trastuzumab. L'invention concerne en particulier la mise en oeuvre de cette méthode avec des échantillons de tissus issus de patients dont le résultat de la détermination de l'expression de HER2 par immunohistochimie s'était révélé négatif (patients Herceptest™-négatifs)

## Etat de la Technique

Le récepteur HER2 (acronyme anglais de « human epidermial growth factor receptor 2 ») est une glycoprotéine transmembranaire de 185 kDa qui appartient à la famille des récepteurs HER à activité tyrosine kinase. En 1987, Slamon et al ont découvert que l'amplification du gène HER2 touche environ un tiers des patients atteints de cancers du sein et est corrélée à un mauvais pronostic vital (Slamon DJ, Clark GM, Wong SG, Levin WJ, Ullrich A, et al. (1987) Science 235: 177-182). Des études ultérieures *in vitro* et *in vivo* ont permis de caractériser les conséquences biologiques de cette anomalie moléculaire, et ont montré que HER2 est un oncogène favorisant la croissance tumorale, l'angiogenèse et le développement de métastases. Il a alors été émis l'hypothèse que l'inhibition de HER2 pourrait être une stratégie thérapeutique efficace pour le traitement des tumeurs surexprimant cette protéine. Cela a conduit à l'élaboration du trastuzumab, un anticorps monoclonal anti-HER2 recombinant humanisé, qui a démontré son efficacité clinique chez les patients atteints de tumeurs du sein surexprimant la protéine HER2, que ce soit dans un contexte métastatique ou adjuvant.

Parallèlement, des tests théranostiques ont été élaborés pour évaluer l'expression de la protéine HER2 afin d'identifier les patients qui pourraient bénéficier d'un traitement par l'anticorps thérapeutique anti-HER2 trastuzumab. Dans la pratique clinique de routine, la quantification de l'expression de HER2 est devenue obligatoire pour la sélection des patients éligibles à ce traitement. Habituellement, le test HER2 est effectué par coloration par immunohistochimie (IHC, par exemple avec test le commercialisé par la société Dako sous la dénomination « Herceptest™ ») qui permet de détecter une surexpression de la protéine HER2, ou par hybridation fluorescente in situ (FISH, acronyme anglais de « fluorescent in situ hybridization »), pour détecter une amplification du gène HER2. L'approche IHC est semi-quantitative et permet de classer les patients sur une échelle de IHC 0 (négatif) à IHC 3 + (fortement positive). Ce classement est basé sur le pourcentage de cellules colorées malignes et le degré de coloration de la membrane de ces cellules. Le test Herceptest™ est considéré comme négatif si les patients sont classés 0, 1+ ou 2+/FISH- et seuls les patients dont le résultat est 3+ ou 2+/FISH+ sont éligibles au traitement par trastuzumab.

Il est connu que l'activité de la protéine HER2 passe par sa dimérisation, notamment par la formation d'homodimères HER2-HER2. Des auteurs se sont ainsi intéressés à la présence des dimères HER2-HER2 chez des patients Herceptest™-positifs et traités par trastuzumab. Il a été par exemple montré dans des études rétrospectives que les patients ayant des taux élevés de dimères Her2-Her2 présentaient une survie globale et une durée de progression de la maladie plus longues que les autres patients lorsqu'ils étaient traités avec trastuzumab (Gosh Cancer Res 2011, Desmet et al Diagn Mol patho 2009).

La demande de brevet WO2009086197 est relative notamment à une méthode visant à déterminer si des patients sont éligibles à un traitement par un anticorps anti-HER2 ou bien pour prédire l'efficacité d'un tel traitement, cette méthode reposant sur la quantification de HER2 ou des homodimères HER2-HER2. Cette méthode est uniquement destinée à être mise en oeuvre sur des patients dont les tests d'expression par IHC ou d'amplification par FISH étaient positifs.

Les approches conventionnelles pour la détection de dimères de HER2 sont basées sur des techniques d'immunoprécipitation et de réticulation chimiques qui ont un faible débit et ne conviennent pas à une utilisation dans un contexte clinique, car elles nécessitent une grande quantité de protéines.

D'autres méthodes ont été proposées, notamment par la société Monogram Biosciences qui a mis au point le test VeraTag™ (Desmedt C et al. 2009 Diagn Mol Pathol 18: 22-29, WO2009086197) pour quantifier les protéines et les dimères de protéines HER dans des échantillons de tissus FFPE. Cette technique, également connue sous la dénomination « eTag » est basée sur l'utilisation de deux anticorps spécifiques de la ou des molécules à détecter, l'un étant couplé via une liaison sensible à l'oxydation avec un marqueur électrophorétique fluorescent, et l'autre étant conjugué à une entité capable de générer de l'oxygène singulet. Lorsque les deux anticorps sont à proximité l'un de l'autre, l'oxygène singulet généré par illumination de l'échantillon va entraîner la libération du marqueur électrophorétique. Les marqueurs libérés sont ensuite séparés par électrophorèse. Cette technique relativement lourde nécessite que les laboratoires d'analyse clinique envoient leurs échantillons à la société Monogram pour analyse.

Le dosage commercialisé sous la dénomination Duolink™ permet la détection de dimères Her2-Her2 par

l'utilisation d'anticorps couplés à des oligonucléotides. Lorsque les anticorps sont séparés par moins de 40 nm, une succession complexe d'étapes d'hybridations et d'amplification conduit à la visualisation des dimères par observation par microscopie de la fluorescence générée *in situ.*

Gaborit et al (J Biol Chem. 2011 Apr 1;286(13):11337-45) ont notamment décrit la détection de dimères Her2-Her2 à la surface de cellules intactes par une approche TR-FRET, c'est-à-dire par la détection en temps résolu de la fluorescence émise lorsque deux molécules fluorescentes compatibles, chacune liée à un anticorps anti-HER2, sont à proximité l'une de l'autre. Cette méthode n'est pas adaptée à une mise en oeuvre sur tissu, dans un contexte clinique.

Malgré les progrès réalisés dans la prise en charge thérapeutique des patients atteints de cancers du sein, ceux ayant été jugés non-éligibles à un traitement de type trastuzumab (par exemple les patients dont les tests Herceptest™ ou FISH étaient négatifs) et en échec thérapeutique après chimiothérapie hormonale se retrouvent malheureusement dans une impasse thérapeutique. L'invention permet d'offrir à ces patients une nouvelle alternative de traitement.

## Description des figures

La Figure 1 représente la probabilité de survie globale en fonction du temps pour des patients atteints de tumeur du sein qui étaient Herceptest™-négatifs et ER-positifs.

La Figure 2 représente la probabilité de survie sans maladie en fonction du temps pour des patients atteints de tumeur du sein qui étaient Herceptest™-négatifs et ER-positifs.

## Description de l'invention

Les inventeurs ont découvert qu'une approche TR-FRET pour détecter et quantifier les homodimères HER2-HER2 dans des échantillons de tissu de patients dont le résultat de la détermination de l'expression de HER2 par immunohistochimie s'était révélé négatif (patients « IHC négatifs » et en particulier Herceptest™-négatifs), permet de révéler de nouveaux patients éligibles à un traitement par un anticorps spécifique de Her2, de type trastuzumab.

Dans un premier aspect, l'invention concerne une méthode (*ex vivo*) de détermination de la susceptibilité d'un patient atteint d'un cancer à répondre à un traitement thérapeutique basé sur l'administration d'un anticorps spécifique de la protéine HER2, cette méthode comprenant la quantification de dimères HER2-HER2 dans un échantillon de tissu dudit patient,

- ladite quantification étant effectuée par la mesure de la fluorescence émise en temps résolu par un couple de partenaires de FRET mis en contact avec ledit échantillon, le premier membre de ce couple étant lié de manière directe ou indirecte à un premier ligand capable de se lier à un domaine de la protéine HER2, le second membre de ce couple étant lié de manière directe ou indirecte à un second ligand capable de se lier au même domaine de la protéine HER2 que le premier ligand, et
- le patient étant un patient dont le résultat de la détermination de l'expression de HER2 par immunohistochimie s'était révélé négatif en particulier le patient est un patient Herceptest™ -négatif.

Par « *couple de partenaires de FRET* » on entend un couple constitué d'un composé fluorescent donneur d'énergie (ci-après « composé fluorescent donneur ») et d'un composé accepteur d'énergie (ci-après « composé accepteur »); lorsqu'ils sont à proximité l'un de l'autre et lorsqu'ils sont excités à la longueur d'onde d'excitation du composé fluorescent donneur, ces composés émettent un signal de FRET (acronyme de l'expression anglaise « Förster Resonance Energy Transfer »).

Par *« signal de FRET »* on entend tout signal mesurable représentatif d'un FRET entre un composé fluorescent donneur et un composé accepteur. Un signal de FRET peut donc être une variation de l'intensité ou de la durée de vie de luminescence du composé fluorescent donneur ou du composé accepteur lorsque ce dernier est fluorescent. Lorsque le signal de FRET est mesuré en temps résolu (ce qui est en général le cas lorsque des chélates ou cryptates de terres rares sont utilisés), on parle de TR-FRET (acronyme de « time-resolved » FRET).

Par «échantillon de tissu », on entend de manière préférée un échantillon de tissu solide prélevé sur un patient, et de préférence un extrait de tissu tumoral. Cet échantillon de tissu est utilisé de préférence sous forme de sections de 10 à 50 $\mu$m d'épaisseur. Ces sections sont préparées selon les techniques classiques bien connues de l'homme du métier. Elles peuvent consister en particulier en la fixation de l'échantillon par un traitement avec du formaldéhyde, son inclusion dans de la paraffine, en particulier sous forme de blocs qui peuvent être ensuite découpés au microtome (de préférence avec une épaisseur d'environ 10 à 30 $\mu$m). Le traitement de ces sections au xylène pour enlever la paraffine, leur rinçage à l'éthanol puis à l'eau sont également des techniques connues de l'homme du métier, de même que la régénération des épitopes par la technique HIER (acronyme de l'expression anglaise « heat induced epitope recovery »).

De manière alternative, la méthode selon l'invention peut également être mise en oeuvre sur des cryosections, de préférence de 20 à 50 μm d'épaisseur, également préparées selon les techniques classiques.

Par « quantification » on entend l'obtention d'un signal dont la valeur dépend de la quantité de dimères HER2-HER2 présents dans l'échantillon. Il est intéressant de noter que selon l'invention, il est possible mais pas nécessaire de comparer la valeur représentant la quantité de dimères de HER2 avec une valeur seuil, celle observée chez des patients de référence, et au-delà de laquelle les patients testés sont éligibles à un traitement par un anticorps anti-HER2. Néanmoins et de manière assez surprenante, il a été déterminé que la seule présence des dimères de HER2 est corrélée à un pronostic négatif et rend donc ces patients éligibles à un traitement par un anticorps anti-HER2. Dans tous les cas, cela rend la méthode selon l'invention très pratique pour le personnel hospitalier puisqu'elle lui permet de conclure très facilement quant à l'éligibilité des patients à ce type de traitement, soit parce que des dimères de HER2 sont détectés, soit parce que la quantité de dimères détectée est supérieurs à une valeur seuil déterminée chez des patients de référence.

La méthode selon l'invention est particulièrement avantageuse lorsqu'elle est mise en oeuvre avec un échantillon de tissu provenant d'un patient ayant fait l'objet d'une chimiothérapie hormonale (patients dits « ER+ », positifs pour l'expression du récepteur de l'estrogène), en particulier lorsque cette approche n'a pas entraîné d'amélioration de son état, puisqu'elle permet de lui offrir une nouvelle alternative thérapeutique, et cela même si le résultat de la détermination de l'expression de HER2 par immunohistochimie (Herceptest™) s'était révélé négatif, c'est-à-dire que les échantillons provenant de ces patients avaient été classés avec un score de 0, 1 + ou 2+/FISH- à ce test.

Dans une mise en oeuvre préférée, le premier et le second ligand sont des anticorps ou des aptamères, et sont spécifiques du même épitope localisé dans la partie extracellulaire ou intracellulaire de la protéine HER2, et ces ligands sont, dans une mise en oeuvre particulièrement avantageuse, identiques. Les anticorps ou aptamères dont l'épitope est situé dans la partie extracellulaire de HER2 sont préférés. Le terme « anticorps » doit ici être pris au sens large et comprend toute protéine de la famille des Immunoglobulines ou bien comportant un domaine d'une immunoglobuline, ainsi qu'un site de liaison spécifique à la protéine d'intérêt. Les anticorps peuvent donc être des fragments Fab, Fab', des anticorps simple chaîne, des domaines variables de chaînes lourdes ou légères d'immunoglobulines. L'homme du métier est à même de fabriquer des anticorps spécifiques de la protéine HER2 en utilisant les techniques classiques. Des anticorps anti-HER2 sont également disponibles dans le commerce.

Par ailleurs, il est souhaitable que les concentrations finales de premier et second ligand dans le milieu d'incubation soient, de manière optimale, supérieures ou égales à 10 nM, de préférence comprises dans la gamme de 10 à 150 nM, de préférence encore dans la gamme de 20 à 80 nM, et de manière particulièrement préférée dans la gamme de 30 à 60 nM. Par concentration finale, on entend la concentration de ces composés dans le milieu d'incubation une fois que tous les réactifs ont été introduits dans ce milieu. Ces domaines de concentrations sont notablement plus élevés que les concentrations habituellement utilisées dans les dosages de type TR-FRET, dans lesquels les concentrations finales de ligands fluorescents sont de l'ordre du nanomolaire, c'est à dire inférieures à 10 nM.

La quantification des dimères HER2-HER2 comprend préférentiellement les étapes suivantes :

(i) mise en contact de l'échantillon de tissu avec le couple de partenaires de FRET liés aux ligands de HER2;
(ii) lavage de l'échantillon de tissu ;
(iii) mesure du signal de FRET émis par le milieu de mesure.

Il peut être intéressant de normaliser le signal de FRET par la quantité de matériel biologique présent dans le milieu de mesure. La méthode selon l'invention comprend ainsi, dans une mise en oeuvre particulière, l'incubation de l'échantillon de tissu avec un agent de marquage émettant un signal proportionnel à la quantité de matériel biologique présent dans l'échantillon. De manière préférée, cet agent de marquage est un agent de marquage fluorescent de l'ADN (par exemple le Hoechst 33342), ajouté à l'échantillon préalablement à l'étape de lavage, et le signal de FRET sera normalisé par le signal correspondant à la fluorescence de cet agent de marquage.

La normalisation peut également être effectuée en utilisant d'autres types d'agents de marquage, en particulier :

- des composés fluorescents marqueurs des mitochondries, tels que le Mitotracker Orange, le Mitotracker Green, ou encore la rhodamine 123. Ces composés sont disponibles dans le commerce et les protocoles de marquage de cellules avec ces composés sont également connus. Ils peuvent être utilisés selon l'invention avec un protocole similaire au cas où l'agent de marquage est un agent intercalant ;
- des composés fluorescents, tels que le chlorure de dansyle ou le NBD (nitrobenzoxadiazole) : ces composés, également disponibles dans le commerce, se lient aux fonctions amines, notamment des protéines. Y.Uratani et al. décrivent un protocole de marquage de cellules avec le chlorure de dansyle (Journal of Bacteriology 1982 p. 523-528). Une fois les cellules marquées, l'invention peut être mise en oeuvre avec un protocole similaire au cas où l'agent de marquage est un agent intercalant ;
- des composés fluorescents qui s'accumulent dans les membranes lipidiques, tels que le Filipin : ce composé est

disponible dans le commerce et son utilisation pour marquer des cellules est connue. Il peut être utilisé selon l'invention avec un protocole similaire à celui du cas ou l'agent de marquage est un agent intercalant.

Enfin, il est avantageux d'inclure dans la méthode de quantification des dimères de HER2 une étape visant à homogénéiser cet échantillon sous forme de lysat cellulaire, et cela préalablement ou postérieurement à l'introduction des composés fluorescents dans le milieu de mesure. Cette étape est de préférence effectuée postérieurement à l'introduction des composés fluorescents dans le milieu d'incubation (premier ligand, second ligand et éventuellement agent de marquage), et préalablement à la mesure du signal de FRET. Un tel traitement peut être mécanique, et peut être choisi parmi : l'application d'ultrasons (sonication), des cycles de congélation / décongélation, l'utilisation de broyeurs mécaniques, éventuellement conjointement à l'utilisation de tampon de lyse hypotonique ou de tampon de lyse contenant des détergents comme le tampon RIPA.

De préférence et pour assurer la sensibilité de la méthode de quantification par TR-FRET, le composé donneur est un chélate ou un cryptate de terre rare, en particulier un chélate ou un cryptate d'europium ou de terbium, et le composé accepteur est choisi parmi les allophycocyanines, les rhodamines, les cyanines, les squaraines, les coumarines, les proflavines, les acridines, les fluorescéines, les dérivés du boron-dipyrrométhène, les fluorophores connus sous la dénomination « Atto », les fluorophores connus sous la dénomination «DY», les composés connus sous la dénomination « Alexa » et le nitrobenzoxadiazole.

***Marquages des ligands ou anticorps par des composés donneurs ou accepteurs d'énergie***

Les ligands peuvent être marqués par les fluorophores de manière directe (covalente) ou indirecte. Le marquage direct est préféré.

Le marquage direct d'un ligand ou d'un anticorps par un composé donneur ou accepteur fluorescent est effectué par les techniques classiques de conjugaison faisant appel à l'utilisation de groupes réactifs. Les composés donneurs ou accepteurs fluorescents sont généralement commercialisés sous forme « fonctionnalisée », c'est-à-dire qu'ils portent un groupe réactif susceptible de réagir avec un groupe fonctionnel présent sur le composé à marquer, en l'occurrence, le ligand.

Typiquement, le groupe réactif présent sur le composé fluorescent donneur ou accepteur est un groupe électrophile ou nucléophile qui peut former une liaison covalente lorsqu'il est mis en présence d'un groupe nucléophile ou électrophile approprié, respectivement. A titre d'exemples, les couples de groupes électrophiles/nucléophiles et le type de liaison covalente formée lorsqu'ils sont mis en présence sont listés ci-dessous :

| **Groupe électrophile** | **Groupe nucléophile** | Type de liaison |
|---|---|---|
| acrylamide | thiol | thioéther |
| halogénure d'acyle | amine/aniline | carboxamide |
| aldéhyde | amine/aniline | imine |
| aldéhyde ou cétone | hydrazine | hydrazone |
| aldéhyde ou cétone | hydroxylamine | oxime |
| alkylsulfonate | thiol | thioéther |
| anhydride | amine/aniline | carboxamide |
| halogénure d'aryle | thiol | thiophénol |
| halogénure d'aryle | amine | arylamine |
| aziridine | thiol | thioéther |
| carbodiimide | acide carboxylique | N-acylurée ou anhydride |
| ester activé* | amine/aniline | carboxamide |
| haloacétamide | thiol | thioéther |
| halotriazine | amine/aniline | aminotriazine |
| imido ester | amine/aniline | amidine |
| isocyanate | amine/aniline | urée |
| isothiocyanate | amine/aniline | thiourée |

(suite)

| **Groupe électrophile** | **Groupe nucléophile** | Type de liaison |
|---|---|---|
| maléimide | thiol | thioéther |
| . sulfonate ester | amine/aniline | alkylamine |
| halogénure de sulfonyle | amine/aniline | sulfonamide |

* : par ester activé, on entend des groupes de formule COY, ou Y est :
- un groupe partant, choisi parmi les groupes succinimidyloxy ($-OC_4H_4NO_2$), sulfo-succinimidyloxy ($-OC_4H_3NO_2-SO_3H$) ;
- un groupe aryloxy substitué ou non par au moins un substituant électrophile tel que les groupes nitro, fluoro, chloro, cyano, trifluorométhyle, formant ainsi un aryle ester activé ;
- un acide carboxylique activé par un groupe carbodiimide, formant un anhydride -OCORa ou -OCNRaNHRb, dans lesquels Ra et Rb sont identiques ou différents et sont choisis parmi les groupes alkyle en $C_1$-$C_6$, perfluoroalkyle en $C_1$-$C_6$, alkoxy en $C_1$-$C_6$, cyclohexyle ;
- le 3-diméthylaminopropyle, ou le N-morpholinoéthyle.

Les composés fluorescents donneurs et accepteurs disponibles dans le commerce comportent généralement une fonction maléimide ou un ester activé, le plus souvent par un groupe NHS (N-hydroxysuccinimidyle), qui réagissent avec les groupes thiol et amines respectivement et peuvent donc être utilisés pour le marquage d'anticorps. Les anticorps marqués sont caractérisés par le rapport molaire final (RMF) qui représente le nombre moyen de molécules de marqueur greffés au ligand.

Lorsque le ligand est de nature protéique, il peut être avantageux d'utiliser l'un des groupes fonctionnels naturellement présent dans les protéines : le groupe amino terminal, le groupe carboxylate terminal, les groupes carboxylates des acides aspartique et glutamique, les groupes amines des lysines, les groupes guanidines des arginines, les groupes thiols des cystéines, les groupes phénols des tyrosines, les cycles indoles des tryptophanes, les groupes thioéthers des méthionines, les groupes imidazoles des histidines.

Si le ligand ne comporte pas de groupe fonctionnel à l'état naturel, de tels groupes peuvent être introduits. Des méthodes d'introduction de groupes fonctionnels sont notamment décrites dans C. Kessler, Nonisotopic probing, Blotting and Sequencing, 2nd édition, L.J. Kricka (1995), Ed. Academic press Ltd., Londres, p. 66-72.

Une autre approche pour le marquage d'un ligand par un composé fluorescent consiste à introduire un groupe réactif dans le ligand, par exemple un groupe NHS ou un groupe maléimide, et de le mettre en présence d'un fluorophore portant un groupe fonctionnel qui réagira avec le groupe réactif pour former une liaison covalente.

Il est important de vérifier que le ligand marqué conserve une affinité suffisante pour son récepteur ; cela peut être contrôlé de manière simple par des expériences de liaison classiques, permettant de calculer la constante d'affinité du ligand marqué pour le récepteur.

Le ligand peut également être marqué par un composé fluorescent de manière indirecte, par exemple en introduisant dans le milieu d'incubation un anticorps dit « secondaire », lui-même lié de manière covalente à un composé fluorescent, cet anticorps reconnaissant de manière spécifique le ligand ou bien un haptène présent sur ce ligand (tel qu'un groupe dinitrophényle, dogoxigénine, la fluorescéine, les étiquettes FLAG, c-myc, 6-HIS). Lorsque le ligand est un anticorps, l'anticorps secondaire peut être un anticorps anti-espèce.

Un autre moyen de marquage indirect très classique consiste à fixer de la biotine sur le ligand à marquer, puis d'incuber ce ligand biotinylé en présence de streptavidine marquée par un fluorophore. Le marquage de ligand par la biotine relève des connaissances générales de l'homme du métier, et la société Cisbio Bioassays commercialise par exemple de la streptavidine marquée avec le fluorophore dont la dénomination commerciale est « d2 » (ref 610SADLA).

***Couples de partenaires de FRET***

Les couples de partenaires de FRET sont de préférence constitués par un composé fluorescent donneur et un composé fluorescent accepteur d'énergie.

Le FRET est défini comme un transfert d'énergie non radiatif résultant d'une interaction dipôle- dipôle entre un donneur et un accepteur d'énergie. Ce phénomène physique nécessite une compatibilité énergétique entre ces molécules. Cela signifie que le spectre d'émission du donneur doit recouvrir, au moins partiellement, le spectre d'absorption de l'accepteur. En accord avec la théorie de Förster, le FRET est un processus qui dépend de la distance séparant les deux molécules, donneur et accepteur : lorsque ces molécules sont à proximité l'une de l'autre, un signal de FRET sera émis.

La sélection du couple fluorophore donneur / accepteur pour obtenir un signal de FRET est à la portée de

l'homme du métier. Des couples donneur-accepteur utilisables pour étudier les phénomènes de FRET sont notamment décrits dans l'ouvrage de Joseph R. Lakowicz (Principles of fluorescence spectroscopy, 2nd edition, Kluwer academic/plenum publishers, NY (1999)), auquel l'homme du métier pourra se référer.

Les composés fluorescents donneurs d'énergie à durée de vie longue (> 0,1 ms, de préférence comprise entre 0,5 et 6 ms), en particulier les chélates ou cryptates de terres rares sont avantageux puisqu'ils permettent d'effectuer des mesures en temps résolu, c'est-à-dire de mesurer des signaux de TR-FRET en s'affranchissant du phénomène d'auto-fluorescence émis par le milieu de mesure. Ils sont pour cette raison et de manière générale préférés pour la mise en oeuvre de la méthode selon l'invention.

Les complexes de dysprosium (Dy3+), de samarium (Sm3+), de neodyme (Nd3+), d'ytterbium (Yb3+) ou encore d'erbium (Er3+) sont des complexes de terres rares également appropriés aux fins de l'invention, mais les chélates et cryptates d'europium (Eu3+) et de terbium (Tb3+) sont particulièrement préférés.

De très nombreux complexes de terres rares ont été décrits et plusieurs sont actuellement commercialisés par les sociétés PerkinElmer, Invitrogen et Cisbio Bioassays.

Des exemples de chélates ou cryptates de terres rares appropriés aux fins de l'invention sont :

- Les cryptates de lanthanides, comportant un ou plusieurs motifs pyridine. De tels cryptates de terre rare sont décrits par exemple dans les brevets EP 0 180 492, EP 0 321 353, EP 0 601 113 et dans la demande internationale WO 01/96 877. Les cryptates de terbium (Tb3+) et d'europium (Eu3+) sont particulièrement appropriés aux fins de la présente invention. Des cryptates de lanthanides sont commercialisés par la société Cisbio Bioassays. On peut citer à titre d'exemple non limitatif les cryptates d'europium de formules ci-dessous (qui peuvent être couplés au composé à marquer via un groupe réactif, ici par exemple un groupe $NH_2$) :

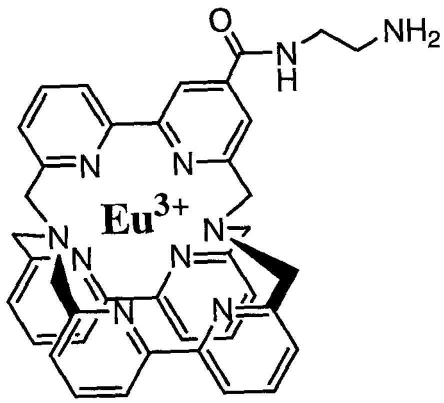

**TrisBiPy-Eu**

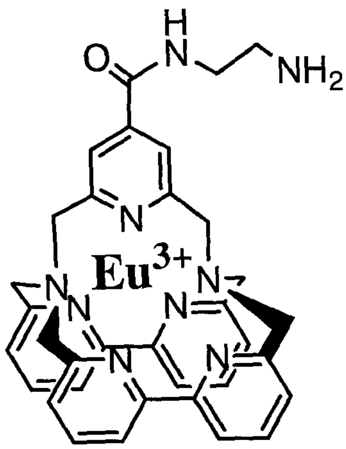

**Py-BiPy-Eu**

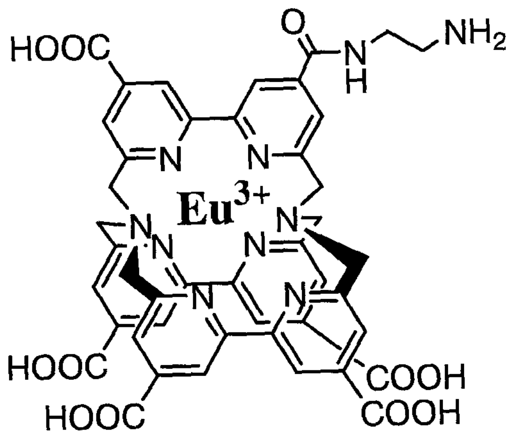

**TrisBiPy-tetraacide-Eu**

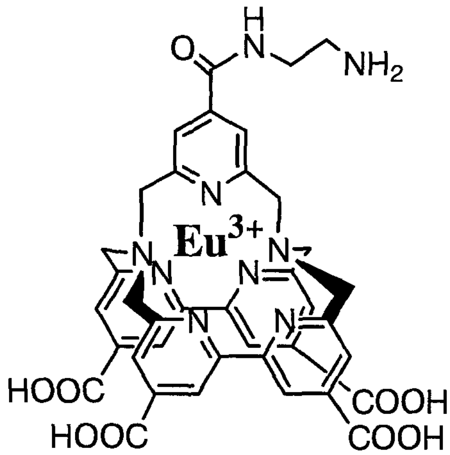

**Py-BiPy-tetraacide-Eu**

- Les chélates de lanthanides décrits notamment dans les brevets US 4 761 481, US 5 032 677, US 5 055 578, US 5 106 957, US 5 116 989, US 4 761 481, US 4 801 722, US 4 794 191, US 4 637 988, US 4 670 572, US 4 837 169, US 4 859 777. Les brevets EP 0 403 593, US 5 324 825, US 5 202 423, US 5 316 909 décrivent des chélates composés d'un ligand nonadenté tel que la terpyridine. Des chélates de lanthanides sont commercialisés par la société PerkinElmer.

- Des complexes de lanthanides constitués d'un agent chélatant, tel que le tétraazacyclododécane, substitué par un chromophore comportant des cycles aromatiques, tels que ceux décrits par Poole R. et al. dans Biomol. Chem, 2005, 3, 1013-1024 "Synthesis and characterisation of highly emissive and kinetically stable lanthanide complexes suitable for usage in cellulo", peuvent également être utilisés. On peut aussi utiliser les complexes décrits dans la demande WO 2009/10580.

- Les cryptates de lanthanides décrits dans les brevets EP 1 154 991 et EP 1 154 990 sont également utilisables.

- Le cryptate de terbium de formule ci-dessous (qui peut être couplé à un composé à marquer via un groupe réactif, ici par exemple un groupe $NH_2$) :

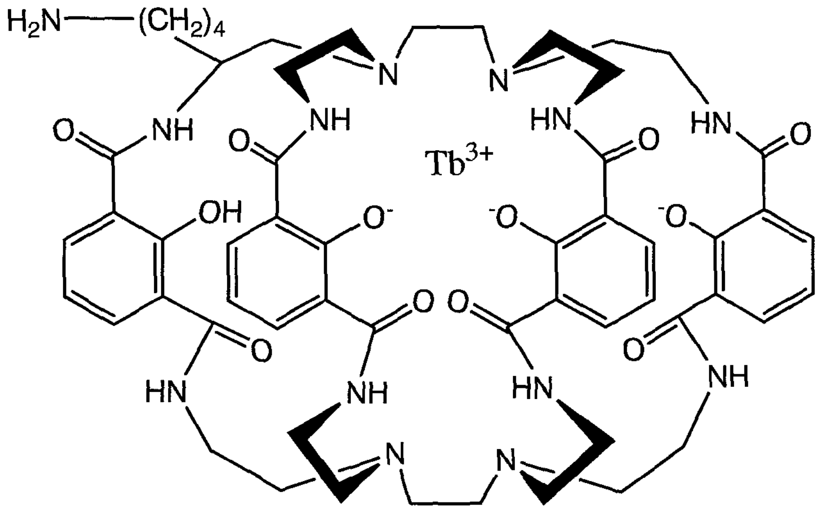

et dont la synthèse est décrite dans la demande internationale WO 2008/063721 (composé 6a page 89).

- Le cryptate de terbium Lumi4-Tb de la société Lumiphore, commercialisé par Cisbio Bioassays.
- Le quantum dye de la société Research Organics, de formule ci-dessous (qui peut être couplé au composé à marquer via un groupe réactif, ici NCS) :

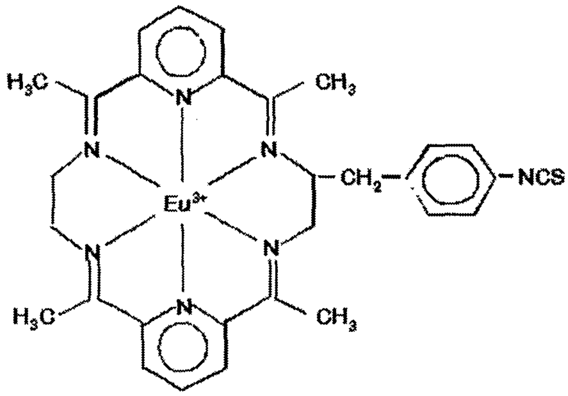

- Les chélates de ruthénium, en particulier les complexes constitués d'un ion ruthénium et de plusieurs bipyridines comme le ruthénium(II) tris(2,2'-bipyridine).
- Le chélate de terbium DTPA-cs124 Tb, commercialisé par la société Life technologies de formule ci-dessous (qui peut être couplé au composé à marquer via un groupe réactif R) et dont la synthèse est décrite dans le brevet américain US 5 622 821.

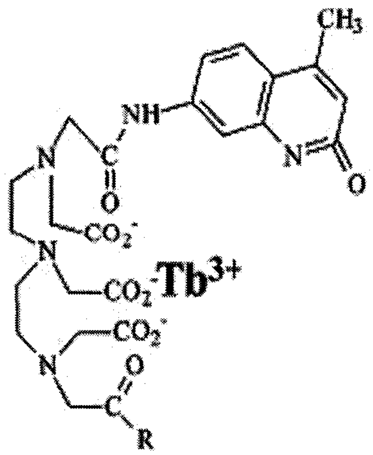

- Le chélate de terbium de formule ci-dessous et décrit par Latva et al (Journal of Luminescence 1997, 75: 149-169) :

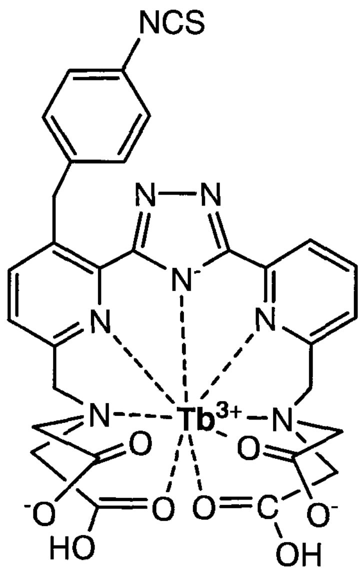

Tb-W14016

De manière particulièrement avantageuse, le composé fluorescent donneur est choisi parmi : un cryptate d'europium; un chélate d'europium ; un chélate de terbium ; un cryptate de terbium ; un chélate de ruthénium ; et un quantum dye ; les chélates et les cryptates d'europium et de terbium étant particulièrement préférés.

Les complexes de dysprosium (Dy3+), de samarium (Sm3+), de néodyme (Nd3+), d'ytterbium (Yb3+) ou encore d'erbium (Er3+) sont également des complexes de terres rares appropriés aux fins de l'invention.

Les composés fluorescents accepteurs peuvent être choisis dans le groupe suivant : les allophycocyanines, en particulier celles connues sous la dénomination commerciale XL665 ; les molécules organiques luminescentes, telles que les rhodamines, les cyanines (comme par exemple Cy5), les squaraines, les coumarines, les proflavines, les acridines, les fluorescéines, les dérivés du boron-dipyrrométhène (commercialisés sous la dénomination « Bodipy »), les fluorophores connus sous la dénomination « Atto », les fluorophores connus sous la dénomination «DY», les composés connus sous la dénomination « Alexa », le nitrobenzoxadiazole. Avantageusement, les composés fluorescents accepteurs sont choisis parmi les allophycocyanines, les rhodamines, les cyanines, les squaraines, les coumarines, les proflavines, les acridines, les fluorescéines, les dérivés du boron-dipyrrométhène, le nitrobenzoxadiazole.

Les expressions « les cyanines » et « les rhodamines » doivent être respectivement comprises comme « les dérivés de cyanine » et « les dérivés de rhodamine ». L'homme du métier connaît ces différents fluorophores, disponibles dans le commerce.

Les composés « Alexa » sont commercialisés par la société Invitrogen; les composés « Atto » sont commercialisés par la société Atto-tec ; les composés « DY» sont commercialisés par la société Dyomics ; les composés « Cy » sont commercialisés par la société Amersham Biosciences ; les autres composés sont commercialisés par divers fournisseurs de réactifs chimiques, tels que les sociétés Sigma, Aldrich ou Acros.

Aux fins de l'invention, les dérivés de cyanines ou de la fluoréscéine sont préférés comme composés fluorescents accepteurs.

## EXEMPLE 1

Dans cet exemple, la méthode selon l'invention a été utilisée pour quantifier la présence de dimères HER2-HER2 dans des échantillons de tumeurs issus de patients, en particulier des tumeurs mammaires.

Les fluorophores Lumi4®Tb et d2 (Cisbio bioassays) ont été utilisés comme partenaires de FRET, respectivement donneur et accepteur. La quantification des homodimères HER2-HER2 a été effectuée avec un seul anticorps (trastuzumab, Roche Pharma AG) marqué soit avec Lumi4®Tb soit avec d2.

Des cryosections de tumeur de 50 μm ont été incubées une nuit dans 180 μl de tampon TR-FRET (1X PBS / 10% BSA + cocktail inhibiteur de protéase, Roche, ref. 1836153) contenant 50 nM de chacun des deux conjugués fluorescents. Une coloration de l'ADN a ensuite été effectuée par ajout de 20 μl d'une solution de Hoechst 33342 (Invitrogen) à 0,1 mg/mL et incubation à température ambiante pendant 10 min. Après lavage et centrifugation, les échantillons ont été remis en suspension dans le tampon TR-FRET, soumis à une sonication et transférés dans une microplaque.

Les signaux de fluorescence de Lumi4®Tb et d2 ont été mesurés respectivement à 620 et 665 nm en mode temps résolu (délai 60 μs ; fenêtre 400μs) après excitation à 337 nm en utilisant un fluorimètre Pherastar ® (BMG Labtech). Le signal du Hoechst 33342 a été mesuré en mode fluorescence à 460 nm. Ces signaux ont été corrigés par le bruit de fond selon la formule :

$$F_{corrigé} = F_{échantillon} - F_{bruitdefond},$$

dans laquelle les valeurs de $F_{bruitdefond}$ ont été obtenues en mesurant la fluorescence du tampon de TR-FRET seul.

Par ailleurs pour chaque dosage, les signaux de fluorescence mesurés sur des solutions obtenues par dilution en cascade au ½ (de façon à obtenir une gamme de concentration) à partir d'une solution mère contenant un mélange de 50 nM d'anticorps-Lumi4 ®-Tb et 50 nM d'anticorps - d2 ont été mesurés simultanément avec les échantillons, et le signal obtenu à 665 nm a été mis en relation avec celui mesuré à 620 nm pour chaque concentration d'anticorps. La courbe ainsi obtenue a été utilisée pour calculer la contribution de la fluorescence de Lumi4®-Tb à 665 nm ($F_{665Tb}$) à partir du signal émis par les échantillons à 620 nm. Le signal de TR-FRET a été exprimé de la manière suivante :

$$\Delta F_{665} = F_{665échantillon} - F_{665Tb}$$

Le signal du complexe ADN-Hoechst 33342 mesuré à 460 nm (Exc 350 nm/ Em 460 nm : $F_{460}$) a été utilisé pour normaliser le signal de TR-FRET pour tenir compte de la variabilité de la quantité de matériel biologique présent dans chaque milieu d'incubation et en normalisant à une valeur moyenne de 100 000 unités de fluorescence (FU):

$$\text{TR-FRET}_{normalisé} = (\Delta F_{665} \text{ X } 100\,000)/\ (F_{460})$$

Le signal de TR-FRET normalisé a été exprimé en FU.

*Résultats : Quantification des dimères HER2-HER2*

18 échantillons de tumeurs mammaires ont été obtenus. Les homodimères HER2-HER2 ont été détectés dans 12 (66,7%) de ces 18 échantillons. Dans la plupart de ces échantillons, les niveaux d'expression des dimères étaient faibles à moyens (200 à 2700 FU), sauf pour cinq échantillons qui ont montré des niveaux élevés de dimères HER2-HER2 (plage 12 000 - 32 400 FU). De manière tout à fait surprenante, les homodimères HER2-HER2 ont été observés non seulement dans les échantillons de tumeurs Herceptest™-positives, mais également dans environ la moitié des tumeurs Herceptest™-négatives (Herceptest™ 0, 1+ ou 2+), même si les signaux observés étaient dans ces cas-là environ 50 fois plus bas que ceux mesurés dans les échantillons de tumeurs Herceptest™-positives.

Pour chaque échantillon, le coefficient de variation de la quantification des dimères HER2-HER2 a été calculé dans trois expériences indépendantes et une valeur moyenne inférieure à 25% pour les trois tests a été obtenue.

Pour la première fois, une méthode fiable de quantification de dimères de HER2 permet de quantifier les

dimères HER2-HER2, et cela même chez des patients Herceptest™-négatifs. Elle ne présente pas les inconvénients de la coloration par immunohistochimie, de la technique FISH et peut être mise en oeuvre à l'hôpital, contrairement à la technique de la société Monogram décrite dans la demande de brevet WO2009086197.

## EXEMPLE 2

Les dimères HER2-HER2 ont été quantifiés par la méthode décrite dans l'exemple 1 sur des échantillons congelés de tumeurs de 100 patients atteints d'un cancer du sein. La mesure de signaux de fluorescence normalisée a permis une mesure quantitative des dimères HER2-HER2. La survie des patients sans maladie (« DFS » pour disease-free survival) et la survie globale (« OS » pour overall survival) ont été évaluées pour chaque patient.

*Résultats*: Parmi les 100 patients, 82 étaient IHC-HER2 négatifs (Herceptest™-négatifs), dont 60 patients qui étaient ER-positifs et traités avec une thérapie hormonale. Les échantillons de 55 de ces 60 patients ont pu être exploités. En utilisant des analyses de risques proportionnels de Cox, il a été montré que chez les sujets IHC-HER2 négatifs et ER-positifs la présence de dimères HER2-HER2 était significativement associée à la fois à une survie globale réduite ($p = 0{,}00237$ ; cf. Figure 1) et à une survie sans maladie réduite ($p = 0{,}00011$ ; cf. Figure 2).

La mesure quantitative de l'expression de HER2 et des dimères HER2-HER2 par le procédé de l'invention peut permettre de prévoir l'issue de la maladie chez des sujets atteints de cancers du sein IHC-HER2 négatifs et ER positifs. Ce biomarqueur est donc utile pour identifier les patients dont le traitement hormonal n'est pas suffisamment efficace et qui pourraient bénéficier d'un traitement adjuvant par thérapie anti-HER de type Herceptin™. C'est là un des apports majeur de l'invention que de pouvoir améliorer la sélection des patients susceptibles de pouvoir répondre à ce type de traitement.

## Revendications

1. Méthode *ex vivo* de détermination de la susceptibilité d'un patient atteint d'un cancer à répondre à un traitement thérapeutique basé sur l'administration d'un anticorps spécifique de la protéine HER2, cette méthode comprenant la quantification de dimères HER2-HER2 dans un échantillon de tissu dudit patient, **caractérisée en ce que**

   - ladite quantification est effectuée par la mesure de la fluorescence émise en temps résolu par un couple de partenaires de FRET mis en contact avec ledit échantillon, le premier membre de ce couple étant lié de manière directe ou indirecte à un premier ligand capable de se lier à un domaine de la protéine HER2, le second membre de ce couple étant lié de manière directe ou indirecte à un second ligand capable de se lier au même domaine de la protéine HER2 que le premier ligand, et **en ce que**
   - le patient est un patient dont le résultat de la détermination de l'expression de HER2 par immunohistochimie s'était révélé négatif.

2. Méthode selon la revendication 1, **caractérisée en ce que** le patient a fait l'objet d'une chimiothérapie hormonale

3. Méthode selon l'une quelconque des revendications précédentes, **caractérisée en ce que** le premier ligand et le second ligand sont des anticorps spécifiques du même épitope localisé dans la partie extracellulaire ou intracellulaire de HER2.

4. Méthode selon l'une quelconque des revendications précédentes, **caractérisée en ce que** le premier ligand et le second ligand sont identiques.

5. Méthode selon l'une quelconque des revendications précédentes, **caractérisée en ce que** lesdits premier et second ligands sont introduits dans le milieu d'incubation à une concentration finale supérieure ou égale à 10 nM.

6. Méthode selon l'une quelconque des revendications précédentes, **caractérisée en ce que** la quantification des dimères HER2-HER2 comprend les étapes suivantes :

   (i) mise en contact de l'échantillon de tissu avec le couple de partenaires de FRET liés aux ligands de HER2;
   (ii) lavage de l'échantillon de tissu ;
   (iii) mesure du signal de FRET émis par le milieu de mesure.

7. Méthode selon la revendication 6, **caractérisée en ce que** la quantification des dimères HER2-HER2 comprend une étape d'incubation de l'échantillon de tissu avec un agent de marquage émettant un signal proportionnel à la

quantité de matériel biologique présent dans l'échantillon cette étape étant préalable à l'étape de lavage, et **en ce que** le signal de FRET est normalisé par le signal correspondant à cet agent de marquage.

**8.** Méthode selon la revendication 7, **caractérisée en ce que** l'agent de marquage est un agent de marquage fluorescent de l'ADN, et **en ce que** le signal de FRET est normalisé par le signal correspondant à la fluorescence de cet agent de marquage.

**9.** Méthode selon l'une quelconque des revendications précédentes, **caractérisée en ce qu'**elle comprend une étape d'homogénéisation de l'échantillon de tissu sous forme de lysat cellulaire.

**10.** Méthode selon la revendication 9, **caractérisée en ce que** l'étape d'homogénéisation de l'échantillon de tissu est mise en oeuvre postérieurement à l'introduction des premier et second ligands, et préalablement à la mesure du signal de FRET.

**11.** Méthode selon l'une quelconque des revendications précédentes, **caractérisée en ce que** le composé donneur est un chélate ou un cryptate de terre rare.

**12.** Méthode selon la revendication 11, caractérisée en que le composé donneur est un chélate ou un cryptate d'europium ou de terbium.

**13.** Méthode selon l'une quelconque des revendications précédentes, **caractérisée en ce que** le composé accepteur est choisi parmi les allophycocyanines, les rhodamines, les cyanines, les squaraines, les coumarines, les proflavines, les acridines, les fluorescéines, les dérivés du boron-dipyrrométhène, les fluorophores connus sous la dénomination « Atto », les fluorophores connus sous la dénomination «DY», les composés connus sous la dénomination « Alexa » et le nitrobenzoxadiazole.

**14.** Méthode selon l'une quelconque des revendications précédentes, **caractérisée en ce que** l'un au moins des partenaires de FRET est lié de manière covalente au premier ligand ou au second ligand.

**15.** Méthode selon l'une quelconque des revendications précédentes, **caractérisée en ce que** les partenaires de FRET sont liés de manière covalente au premier ligand et au second ligand.

**16.** Méthode selon l'une quelconque des revendications précédentes, **caractérisée en ce que** le patient est un patient Herceptest™-négatif.

## Patentansprüche

**1.** *Ex* vivo-Verfahren zur Bestimmung der Suszeptibilität eines an einem Krebs erkrankten Patienten, auf eine therapeutische Behandlung, die auf der Verabreichung eines spezifischen Antikörpers des HER2-Proteins basiert, anzusprechen, wobei dieses Verfahren die Quantifizierung von HER2-HER2-Dimeren in einer Gewebeprobe des Patienten umfasst, worin:

- die Quantifizierung durch das Messen der Fluoreszenz, die durch ein mit der Probe in Kontakt gebrachtes Paar von FRET-Partnern zeitaufgelöst ausgesandt wird, vollzogen wird, wobei das erste Glied dieses Paares direkt oder indirekt an einen ersten Liganden gebunden ist, der geeignet ist, an eine Domäne des HER2-Proteins zu binden, wobei das zweite Glied dieses Paares direkt oder indirekt an einen zweiten Liganden gebunden ist, der geeignet ist, an die gleiche Domäne des HER2-Proteins wie der erste Ligand zu binden, und dass
- der Patient ein Patient ist, dessen Ergebnis der Bestimmung der HER2-Expression durch Immunhistochemie sich als negativ herausgestellt hat.

**2.** Verfahren nach Anspruch 1, worin der Patient sich einer hormonellen Chemotherapie unterzogen hat.

**3.** Verfahren nach einem der vorhergehenden Ansprüche, worin der erste Ligand und der zweite Ligand spezifische Antikörper des in dem extrazellulären oder intrazellulären Teil von HER2 lokalisierten gleichen Epitops sind.

**4.** Verfahren nach einem der vorhergehenden Ansprüche, worin der erste Ligand und der zweite Ligand identisch sind.

5. Verfahren nach einem der vorhergehenden Ansprüche, worin der erste und der zweite Ligand in einer Endkonzentration von über oder gleich 10 nM in das Inkubationsmedium eingebracht werden.

6. Verfahren nach einem der vorhergehenden Ansprüche, worin die Quantifizierung der HER2-HER2-Dimere die folgenden Schritte umfasst:

(i) Inkontaktbringen der Gewebeprobe mit dem Paar von an die Liganden von HER2 gebundenen FRET-Partnern,
(ii) Waschen der Gewebeprobe,
(iii) Messen des durch das Messmedium ausgesandten FRET-Signals.

7. Verfahren nach Anspruch 6, worin die Quantifizierung der HER2-HER2-Dimere einen Schritt der Inkubation der Gewebeprobe mit einem Markierungsmittel, welches ein Signal proportional zur Menge an in der Probe vorhandenem biologischem Material aussendet, wobei dieser Schritt vor dem Waschschritt stattfindet, und worin das FRET-Signal durch das Signal, das diesem Markierungsmittel entspricht, normalisiert ist.

8. Verfahren nach Anspruch 7, worin das Markierungsmittel ein DNA-Fluoreszenzmarkierungsmittel ist und worin das FRET-Signal durch das Signal, das der Fluoreszenz dieses Markierungsmittels entspricht, normalisiert ist.

9. Verfahren nach einem der vorhergehenden Ansprüche, worin es einen Schritt zur Homogenisierung der Gewebeprobe in Form von Zelllysat umfasst.

10. Verfahren nach Anspruch 9, worin der Schritt der Homogenisierung der Gewebeprobe nach dem Einbringen des ersten und des zweiten Liganden und vor der Messung des FRET-Signals durchgeführt wird.

11. Verfahren nach einem der vorhergehenden Ansprüche, worin die Donor-Verbindung ein Chelat oder ein Kryptat von Seltene Erde ist.

12. Verfahren nach Anspruch 11, worin die Donor-Verbindung ein Chelat oder ein Kryptat von Europium oder von Terbium ist.

13. Verfahren nach einem der vorhergehenden Ansprüche, worin die Akzeptor-Verbindung aus den Allophycocyaninen, den Rhodaminen, den Cyaninen, den Squarainen, den Cumarinen, den Proflavinen, den Akridinen, den Fluoresceinen, den Derivaten von Boron-Dipyrromethen, den unter der Bezeichnung "Atto" bekannten Fluorophoren, den unter der Bezeichnung "DY" bekannten Fluorophoren, den unter der Bezeichnung "Alexa" bekannten Verbindungen und Nitrobenzoxadiazol ausgewählt ist.

14. Verfahren nach einem der vorhergehenden Ansprüche, worin wenigstens einer der FRET-Partner an den ersten Liganden oder an den zweiten Liganden kovalent gebunden ist.

15. Verfahren nach einem der vorhergehenden Ansprüche, worin die FRET-Partner an den ersten Liganden und an den zweiten Liganden kovalent gebunden sind.

16. Verfahren nach einem der vorhergehenden Ansprüche, worin der Patient ein HercepTest™-negativ-Patient ist.

## Claims

1. An *ex vivo* method for determining the susceptibility of a patient suffering from cancer to respond to a therapeutic treatment based on the administration of an antibody specific for the HER2 protein, this method comprising the quantification of HER2-HER2 dimers in a tissue sample from said patient, wherein:

- said quantification is carried out by time resolved measurement of the fluorescence emitted by a pair of FRET partners brought into contact with said sample, the first member of this pair being directly or indirectly bonded to a first ligand capable of binding to a domain of the HER2 protein, the second member of this pair being directly or indirectly bonded to a second ligand capable of binding to the same domain of the HER2 protein as the first ligand, and in that
- the patient is a patient for whom the result of the determination of HER2 expression by immunohistochemistry

had proved to be negative.

2. The method as claimed in claim 1, wherein the patient has been the subject of hormone chemotherapy.

3. The method as claimed in either one of the preceding claims, wherein the first ligand and the second ligand are antibodies specific for the same epitope located in the extracellular or intracellular part of HER2.

4. The method as claimed in any one of the preceding claims, wherein the first ligand and the second ligand are identical.

5. The method as claimed in any one of the preceding claims, wherein said first and second ligands are introduced into the incubation medium at a final concentration greater than or equal to 10 nM.

6. The method as claimed in any one of the preceding claims, wherein the quantification of the HER2-HER2 dimers comprises the following steps:

(i) bringing the tissue sample into contact with the pair of FRET partners bonded to the HER2 ligands;
(ii) washing the tissue sample;
(iii) measuring the FRET signal emitted by the measuring medium.

7. The method as claimed in claim 6, wherein the quantification of the HER2-HER2 dimers comprises a step of incubating the tissue sample with a labeling agent which emits a signal proportional to the amount of biological material present in the sample, this step being prior to the washing step, and wherein the FRET signal is standardized with respect to the signal corresponding to this labeling agent.

8. The method as claimed in claim 7, wherein the labeling agent is a fluorescent DNA-labeling agent, and wherein the FRET signal is standardized with respect to the signal corresponding to the fluorescence of this labeling agent.

9. The method as claimed in any one of the preceding claims, wherein it comprises a step of homogenizing the tissue sample in the form of a cell lysate.

10. The method as claimed in claim 9, wherein the step of homogenizing the tissue sample is carried out after the introduction of the first and second ligands, and before the measurement of the FRET signal.

11. The method as claimed in any one of the preceding claims, wherein the donor compound is a rare earth chelate or cryptate.

12. The method as claimed in claim 11, wherein the donor compound is a europium or terbium chelate or cryptate.

13. The method as claimed in any one of the preceding claims, wherein the acceptor compound is chosen from allophycocyanins, rhodamines, cyanines, squaraines, coumarins, proflavins, acridines, fluoresceins, boron-dipyrromethene derivatives, fluorophores known under the name "Atto", fluorophores known under the name "DY", compounds known under the name "Alexa" and nitrobenzoxadiazole.

14. The method as claimed in any one of the preceding claims, wherein at least one of the FRET partners is covalently bonded to the first ligand or to the second ligand.

15. The method as claimed in any one of the preceding claims, wherein the FRET partners are covalently bonded to the first ligand and to the second ligand.

16. The method as claimed in any one of the preceding claims, wherein the patient is a Herceptest™-negative patient.

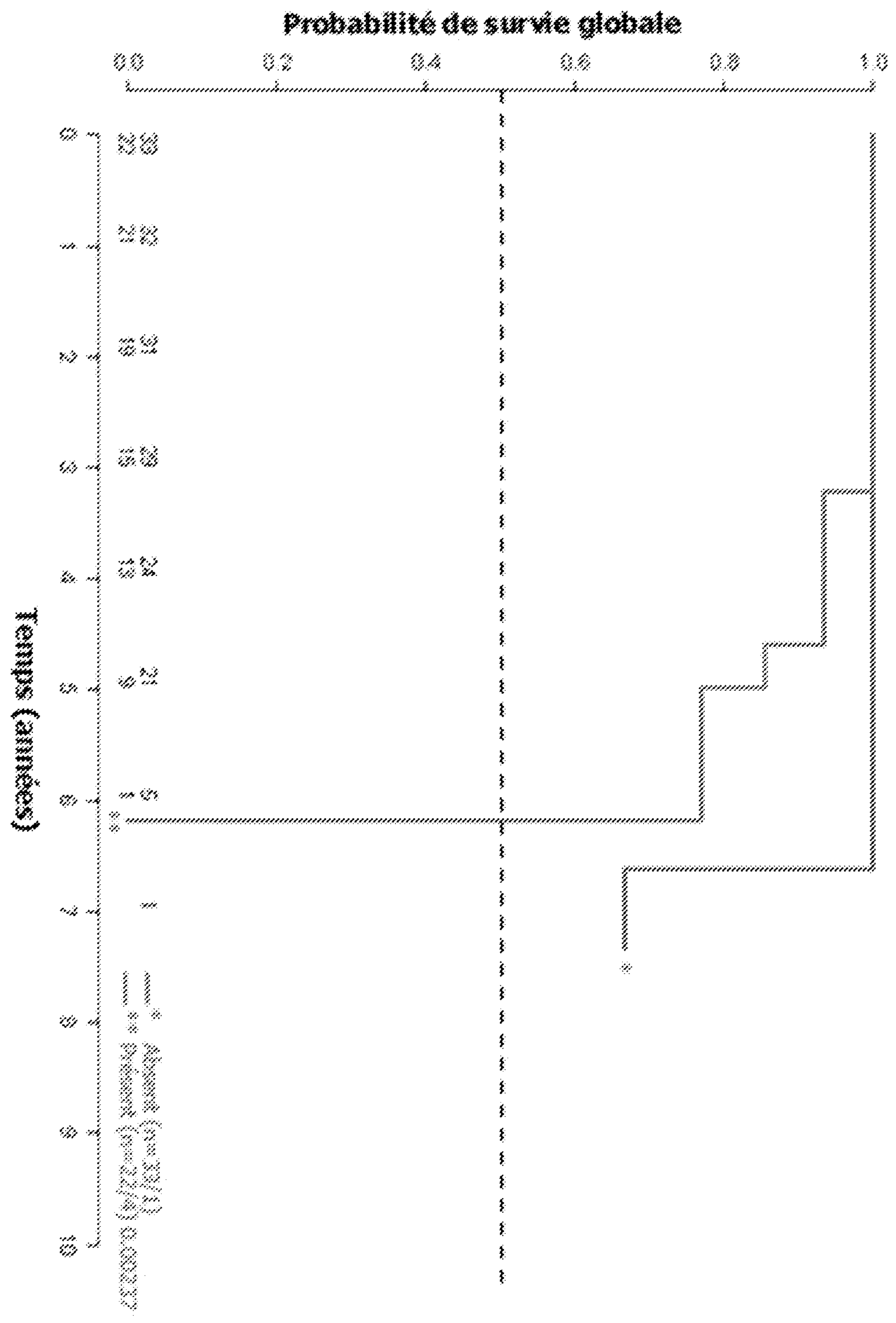


FIG.1

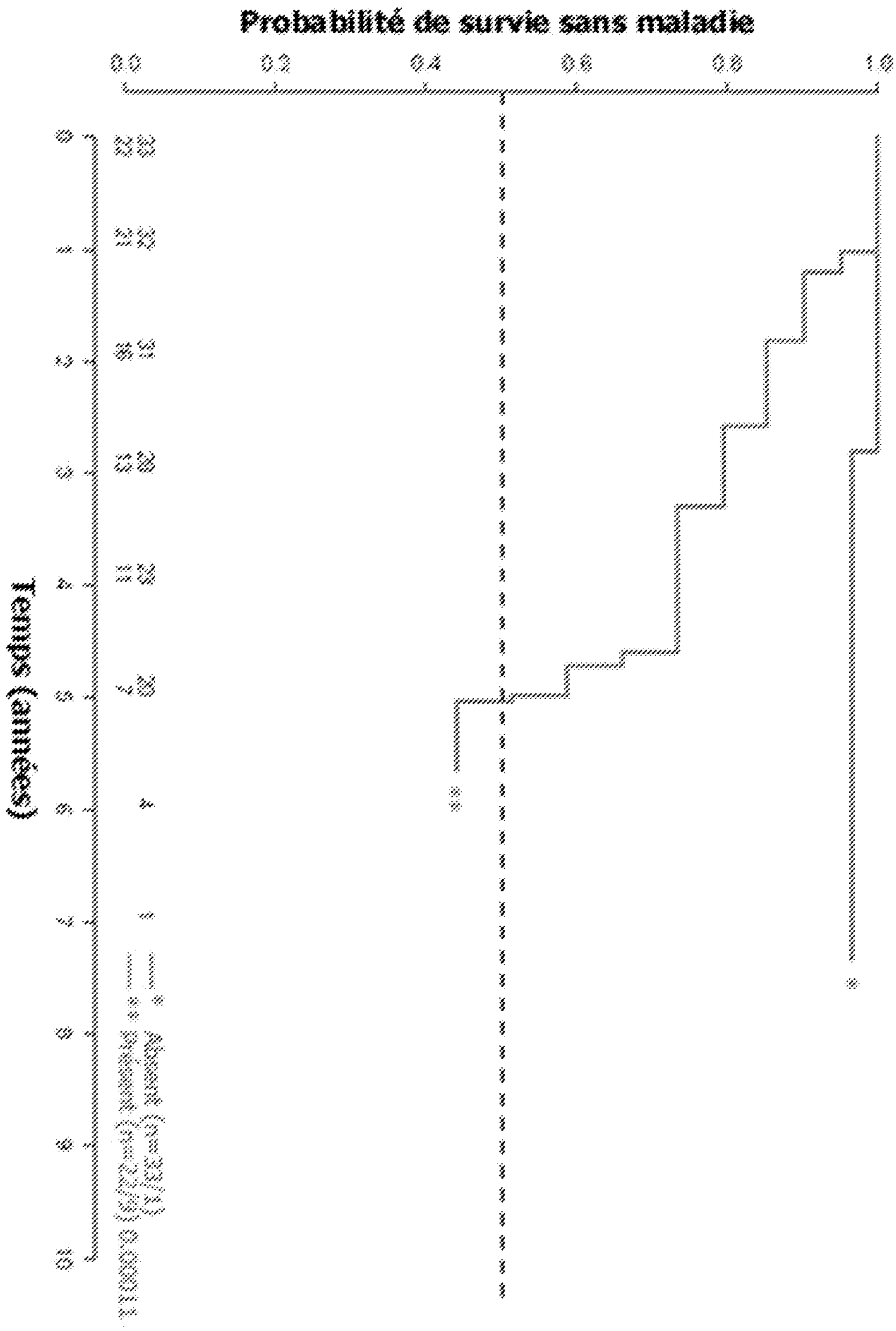


FIG.2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2009086197 A
- EP 0180492 A
- EP 0321353 A
- EP 0601113 A
- WO 0196877 A
- US 4761481 A
- US 5032677 A
- US 5055578 A
- US 5106957 A
- US 5116989 A
- US 4801722 A
- US 4794191 A
- US 4637988 A
- US 4670572 A
- US 4837169 A
- US 4859777 A
- EP 0403593 A
- US 5324825 A
- US 5202423 A
- US 5316909 A
- WO 200910580 A
- EP 1154991 A
- EP 1154990 A
- WO 2008063721 A
- US 5622821 A


### Littérature non-brevet citée dans la description


- **SLAMON DJ ; CLARK GM ; WONG SG ; LEVIN WJ ; ULLRICH A et al.** *Science,* 1987, vol. 235, 177-182
- **GOSH.** *Cancer Res,* 2011
- **DESMET et al.** *Diagn Mol patho,* 2009
- **DESMEDT C et al.** *Diagn Mol Pathol,* 2009, vol. 18, 22-29
- **GABORIT et al.** *J Biol Chem.,* 01 Avril 2011, vol. 286 (13), 11337-45
- **Y.URATANI et al.** protocole de marquage de cellules avec le chlorure de dansyle. *Journal of Bacteriology,* 1982, 523-528
- **C. KESSLER.** Nonisotopic probing, Blotting and Sequencing. Academic press Ltd, 1995, 66-72
- **JOSEPH R. LAKOWICZ.** Principles of fluorescence spectroscopy. Kluwer academic/plenum publishers, 1999
- **POOLE R et al.** Synthesis and characterisation of highly emissive and kinetically stable lanthanide complexes suitable for usage in cellulo. *Biomol. Chem,* 2005, vol. 3, 1013-1024
- **LATVA et al.** *Journal of Luminescence,* 1997, vol. 75, 149-169